# EUROPEAN PATENT APPLICATION

(11) **EP 3 865 560 A1**
(43) Date of publication of application: **18.08.2021**
(21) Application number: 20382116.0
(22) Date of filing: 17.02.2020
(51) Int. Cl.: C11B 1/02, A23D 9/02

(54) **USE OF PHOSPHOLIPASE FOR OLIVE OIL EXTRACTION**

(71) Applicant: Solis Nadal, Daniel Rafael, 08035 Barcelona (ES)
(72) Inventor: Solis Nadal, Daniel Rafael, 08035 Barcelona (ES)
(74) Representative: Durán-Corretjer, S.L.P.

(57) **Abstract**

The present invention relates to the use of an enzyme preparation comprising at least one phospholipase for the extraction of olive oil. The present invention also relates to a method for olive oil extraction comprising a step of addition of an enzyme preparation comprising at least one phospholipase and to enzyme preparations comprising at least one phospholipase and at least one enzyme that degrades the cell wall selected from the group consisting of xylanase, cellulase, pectinase, protease, cutinase, or a mixture thereof.

## Description

The present invention relates to the field of olive oil production, and more particularly, to the use of enzyme preparations comprising phospholipases for olive oil extraction.

Olive oil is one of the most consumed products in the countries of the Mediterranean basin, where 70 % of world production is consumed. For centuries, olive oil has not only been part of the culture of many European countries, where it is considered the basis of Mediterranean cuisine, but it is also recognized as a very healthy foodstuff because of the many benefits derived from its regular use. These benefits are largely related to the high amount of monounsaturated fatty acids in olive oil, but also to the presence of a large number of antioxidant agents, such as tocopherol, phenolic compounds, chlorophyll and carotenoids.

In addition to the wide use of olive oil in the diet, in recent years its use has also been extended to other sectors of the industry, such as the pharmaceutical sector, where it is used, for example, in the treatment of alopecia, paralysis, rheumatism and hypertension, the cosmetic sector, where it is used for example in creams because of its moisturizing and emollient properties, and in other areas such as in the manufacture of soaps, textiles and lubricants.

For these reasons, the international community is becoming more and more interested in olive oil and the methods for its production. In fact, the world consumption of olive oil has increased by 50 % in the last 25 years and, although the production of olive oil is concentrated mainly in the Mediterranean countries (mainly countries of the European Union, Tunisia, Morocco, Syria and Turkey), its consumption in countries such as Japan, Brazil, Russia or Canada has also increased notably in recent decades.

Olive oil is obtained from the fruit of the olive tree, which is the olive. The methods for olive oil extraction, which takes place in an oil mill, has been changing over the years, including mechanical, chemical or physical extraction techniques or combinations thereof. This extraction method varies depending on the type of olive oil that is intended to be obtained.

In general, the olive oil extraction method comprises the following essential steps:
- Collection and preparation of the raw material including selection, cleaning and washing the olives.
- Crushing or grinding the olives, in which different types of press are used until obtaining a paste with a determined granulometry. This paste contains olive oil in the form of small drops, but can also contain crushed olive bones, water and solid vegetable materials.
- Malaxation the olive paste to form larger oil drops and facilitate the subsequent separation of the olive oil.
- Separation of the different components of the olive paste according to their density. The separation is generally carried out by cold centrifugation and usually comprises a first step in which the paste is centrifuged in a horizontal centrifugal decanter, and a second step, in which a vertical centrifuge is used. This two-phase separation process is the most widely used at present and allows the oil to be separated from the remaining waste, which include the "alperujo", a residue that in some cases is recovered as pomace and "alpechín" separately, and the wastewater.
- Storage of olive oil in stainless steel tanks at constant temperature prior to packaging, transport and marketing.

After the grinding, malaxation and separation steps, all carried out at cold temperature), the highest quality olive oil is obtained, that is, virgin and extra virgin olive oil. These oils are characterized by having the best nutritional and organoleptic properties. According to European legislation, it is only possible to consider an olive oil as virgin or extra virgin if its acidity does not exceed 2º and 0.8º respectively, and if said oils have been extracted by process that take place at cold temperature (temperature below 27 ºC), without employing any method that involves the use of high temperatures that can destroy or eliminate some of the most interesting components of the oil.

Therefore, virgin and extra virgin olive oils are those that are extracted only from the first "pressing" of the olive.

However, it is also possible to subject the residues obtained in said first extraction or pressing of virgin and extra virgin olive oil to additional steps to obtain other types of olive oils of inferior quality. Thus, the residues such as "alperujo", can be subjected to a second malaxation step and a second step of cold separation, to recover the oil that may have remained in them. The oils obtained in the second pressing can be of various types, such as the so-called olive oil or "lampante" oil, and which are characterized by having a degree of acidity greater than 2º. In some cases, said oils of inferior quality obtained in the second pressing are mixed with virgin olive oils to decrease their acidity and improve their properties.

It is also possible to subject the residues obtained in both the first and the second pressing to subsequent refining steps to obtain other types of oils, such as pomace oil. In certain cases, said refining steps are essential for the oil to be suitable for consumption. For example, when the olive is not of good quality or is in poor condition, or when there has been a problem during the extraction methods, the oil obtained may have acidity much higher than 2º or have an unpleasant smell and taste, which makes it non adequate for consumption. However, if said oil undergoes additional refining steps, the components that make it non adequate for consumption can be eliminated while improving its nutritional and organoleptic characteristics. The refining steps are usually very aggressive steps that take place at very high temperatures, so they are not allowed under any circumstances in the production of virgin and extra virgin olive oil. Said steps pursue, among other objectives, to decrease the unsaponifiable fraction of the mixture, which allows increasing the physical stability of the oil and favoring its subsequent processing, increasing thus the final yield of the method. Although the refining of said oils makes them suitable for consumption, their nutritional and organoleptic characteristics are much lower than those of virgin and extra virgin olive oil.

In order to evaluate the efficacy of a method for olive oil extraction, the amount of oil obtained per volume of olives is taken into account, and whether said oil has not been altered and, therefore, it maintains its original properties and qualities.

In recent years, producers and researchers have tried to develop new techniques for extracting olive oil by increasing the yield while maintaining oil properties. It is considered that around 25 % of the oil is trapped within the colloidal tissues of the cytoplasm of the olive cells or it forms an emulsion with the vegetable water, which greatly hinders its recovery.

Therefore, one of the main issues when increasing the yield of olive oil extraction is to decrease the percentage of oil that is not recovered, without affecting its nutritional and organoleptic properties.

One of the options that the industry has proposed is the use of enzymes capable of degrading the cell walls of the olive, which are mainly composed of cellulose, pectin, hemicellulose, glycoproteins and lignin. Thus, the addition of exogenous enzymes capable of degrading these walls, such as cellulases, xylinases, pectinases, proteases, or cutinases, could be an option to increase the yields in the extraction of olive oil.

For example, in the document "Use of Enzymatic Preparations to Enhance Olive Oil Extraction and their quality, Al-Rousan et al., J. of Food and Nutr. 2019, Vol. 7, No. 4, 311-318 "it is shown that the use of cellulase and/or pectinase during the malaxation step can increase the yield of the method for olive oil extraction up to 5 % without significantly affecting the acidity, and the peroxide and UV extinction coefficient values. In addition, by using these enzymes it was also possible to increase the levels of antioxidant compounds in the olive oil. This study was carried out in Jordan, and as the authors concluded, the observed yield increase can be considered sufficient to satisfy the market demands in that country.

However, due to the exponential increase in the consumption of olive oil worldwide, there is still a need for more efficient methods, which allow for a much greater increase in yield to satisfy the demand, not only in the food industry, but also in other sectors, in which the use of olive oil has also increased lately.

Surprisingly, the inventors of the present invention have discovered that the use of other enzymes, not related to the degradation of the cell wall, such as the enzymes of the phospholipase family, allows to further increase the yield of the methods for olive oil extraction without affecting oil nutritional and organoleptic properties.

Phospholipases are a family of enzymes that catalyse the hydrolysis of ester bonds in phospholipids, originating fatty acids and other lipophilic substances. This family encompasses type A (A1, A2, A3), type B, type C, type D, and type PL phospholipases. Type C phospholipases include those that come from humans (phosphoinositide phospholipase C) and those that come from bacteria, trypanosomes, or other microorganisms. Phospholipases are enzymes widely used in other industry areas, such ethanol and other biofuels production, cosmetics or the production of bakery or dairy products.

Despite its wide use in these industrial areas, the use of phospholipases in methods for olive oil extraction, and more specifically in the malaxation and / or separation steps, has not been described so far, so the present Invention provides a new use for these enzymes in the olive oil sector.

The high availability of different types of phospholipases on the market and their relatively low cost make the use of said enzymes in the extraction of olive oil very interesting from an economic point of view. In addition, the use of these phospholipases in the extraction of olive oil also results in environmental benefit, since by increasing the yield of the extraction method, waste generation is reduced as well, which are highly polluting since they are difficult to remove.

The present invention relates to the field of olive oil production and to the related extraction steps, such as grinding, malaxation and cold separation. Likewise, the present invention refers to the production of refined olive oils that have been obtained by adding olive oil obtained by cold extraction and does not refer to the field of refined vegetable oils production, among which refined olive oil is included, which has been obtained by other methods taking place at elevated temperatures or in the presence of chemical compounds.

As used herein, the term "comprises" or variations such as "comprise" or "comprising", shall be understood to imply the inclusion of a particular element or groups of elements, but not the exclusion of any other element or group of elements.

Each embodiment of the present invention will be applied *mutatis mutandis* to any other embodiment unless expressly stated otherwise.

As used herein, the terms "virgin olive oil" and "extra virgin olive oil" refer only to those oils that, according to Regulation (EC) No. 640/2008 of the European Commission, can be classified thereby.

As used herein, the term "refined oil" refers to any oil of vegetable origin that has undergone at least one refining step and that said at least one refining step cannot be applicable to obtaining virgin or extra virgin olive oil.

As used herein, the term "phospholipase" refers to any enzyme in the phospholipase family capable of hydrolyzing the ester bonds of phospholipids. A person skilled in the art knows which enzymes are called phospholipases, but by way of example, a non-limiting list is included herein that comprises type A (A1, A2, A3), type B, type C, and type D and PL type phospholipases. Furthermore, type C phospholipases include those that come from humans (phosphoinositide phospholipase C) and those that come from bacteria, trypanosomes, or other microorganisms.

As used herein, the term "enzyme preparation" refers to any preparation, either in solid or liquid form, that comprises an enzyme in an amount sufficient to carry out its biological function. The person skilled in the art has the required information to determine the required quantity of each enzyme in the enzyme preparation of the present invention so that it can carry out its biological function in a specific sample. In the case of the present invention, the term "enzyme preparation" is used to refer to a preparation comprising at least one phospholipase.

In a first aspect, the present invention discloses the use of an enzyme preparation for the extraction of olive oil, characterized in that said enzyme preparation comprises at least one phospholipase. In a preferred embodiment, the present invention discloses the use of an enzyme preparation for the extraction of virgin or extra virgin olive oil, characterized in that said enzyme preparation comprises at least one phospholipase.

In a preferred embodiment of the present invention, said at least one phospholipase is selected from the group consisting of phospholipase A1, phospholipase A2, phospholipase A3, phospholipase B, phospholipase C, phospholipase D, phospholipase PL, phosphoinositide phospholipase C, or a mixture thereof.

The phospholipase enzymes of the present invention can be obtained both from animal or plant extracts, and also by microbiological production. The phospholipases of the present invention can also be obtained from animal extracts such as porcine or bovine pancreas, although other animal extracts can also be used as a source of phospholipases. Other sources of phospholipases are vegetables, such as white cabbages or green cabbages, among others, or some protists, such as trypanosomes. It is also possible to obtain phospholipases by microbiological production in different species of bacteria and fungi, among which *Pseudomonas* spp, *Escerichia* coli, *Arpergillus* spp, *Bacillus* spp *Streptomyces* spp are found, without limitation. Said microbiological production includes both the production of unmodified or natural (wild-type) enzymes of said species, and the production of recombinant proteins.

In a preferred embodiment of the present invention, said at least one phospholipase is of animal, plant or microbiological origin.

In a more preferred embodiment of the present invention, said at least one phospholipase is of animal origin. In another more preferred embodiment of the present invention, said at least one phospholipase is of plant origin. In another more preferred embodiment of the present invention, said at least one phospholipase is of microbiological origin. In another even more preferred embodiment of the present invention, said at least one phospholipase is of recombinant origin.

In a preferred embodiment of the present invention, the enzyme preparation further comprises at least one enzyme that degrades the cell wall. There are different enzymes capable of degrading the cell wall and one skilled in the art knows which are the most suitable for the present invention. In a more preferred embodiment, said at least one enzyme that degrades the cell wall is selected from the group consisting of xylanase, cellulase, pectinase, protease, cutinase, or a mixture thereof. However, other enzymes capable of degrading the cell wall can also be part of the enzyme preparation of the present invention.

In another embodiment, the enzyme preparation of the present invention is in solid or liquid form. Preferably, said enzyme preparation is in the form of a powder, in the form of an aqueous solution, a glycerol solution, a solution in potassium sorbate or in any other solution or buffer known to the person skilled in the art suitable for the enzymes of the present invention. More preferably, said enzyme preparation is in form of a powder or in the form of a glycerol solution.

The enzyme preparation of the present invention can be used in the different steps of the method for olive oil extraction. A person skilled in the art knows well the different steps of said method and can determine in which step or steps the enzyme preparation can be added.

In a preferred embodiment of the present invention, the enzyme preparation is added before or during the grinding step of a method for olive oil extraction. In another preferred embodiment of the present invention, the enzyme preparation is added before or during the malaxation step of a method for olive oil extraction. In another preferred embodiment of the present invention, the method for olive oil extraction is a method for virgin or extra virgin olive oil extraction.

In other embodiments, the method for olive oil extraction of the present invention may comprise second steps of malaxation and separation, which is known to the person skilled in the art as second extraction or second pressing. In such embodiments, the enzyme preparation of the present invention is added before or during any of the malaxation steps, or before or during both malaxation steps.

In the embodiments of the present invention in which more than one grinding step and / or more than one malaxation step are carried out, the enzyme preparation can be added before or during any of said steps or in several of said steps.

The concentration of each enzyme in the enzyme preparation according to the present invention is determined based on the initial concentration each enzyme is available and the final desired concentration in the mixture to which said enzyme preparation is added during the extraction method. In a preferred embodiment of the present invention, the final concentration in the mixture of the at least one phospholipase is between 0.1 % and 20 % by weight, in a more preferred embodiment, between 1 % and 10 % in weight, in a more preferred embodiment, between 2 % and 5 % by weight.

In a second aspect, the present invention discloses a method for olive oil extraction, characterized in that it comprises a step of addition of the enzyme preparation that comprises at least one phospholipase according to the present invention.

In a preferred embodiment, said at least one phospholipase is selected from the group consisting of phospholipase A1, phospholipase A2, phospholipase A3, phospholipase B, phospholipase C, phospholipase D, phospholipase PL, phosphoinositide phospholipase C, or a mixture thereof.

In a preferred embodiment of the present invention, said at least one phospholipase is of animal, plant or microbiological origin.

In a more preferred embodiment, the enzyme preparation of the present invention further comprises at least one enzyme that degrades the cell wall. In another more preferred embodiment, said at least one enzyme that degrades the cell wall is selected from the group consisting of xylanase, cellulase, pectinase, protease, cutinase, or a mixture thereof.

In another embodiment, the enzyme preparation of the present invention is in solid or liquid form. Preferably, said enzyme preparation is in the form of a powder, in the form of an aqueous solution, a glycerol solution, a solution in potassium sorbate or in any other solution or buffer known to the person skilled in the art suitable for the enzymes of the present invention. More preferably, said enzyme preparation is in the form of a powder or in the form of a glycerol solution.

Said enzyme preparation of the present invention can be used in the different steps of the method for olive oil extraction. A person skilled in the art knows well the different steps of said method and can determine in which step or steps the enzyme preparation can be added.

In a preferred embodiment of the method of the present invention, the enzyme preparation is added before or during the grinding step of said method. In another preferred embodiment of the method of the present invention, the enzyme preparation is added before or during the malaxation step of said method. In another preferred embodiment of the present invention, the method for olive oil extraction is a method for virgin or extra virgin olive oil extraction.

In another more preferred embodiment of the method of the present invention, the enzyme preparation is added only during the malaxation step of said method for olive oil extraction. In another more preferred embodiment of the method of the present invention, the enzyme preparation is added only during the malaxation step of said method for virgin or extra virgin olive oil extraction.

In the embodiments of the present invention in which more than one grinding step and / or more than one malaxation step are carried out, the enzyme preparation can be added before or during any of said steps or in several of said steps.

In other embodiments, the method for olive oil extraction of the present invention may comprise second steps of malaxation and separation, which is known to the person skilled in the art as second extraction or second pressing. In such embodiments, the enzyme preparation of the present invention is added before or during any of the malaxation steps, or before or during both malaxation steps.

In another preferred embodiment, the method for olive oil extraction of the present invention is not a method for vegetable oil refining. In another preferred embodiment, said olive oil is not refined oil. In another more preferred embodiment, said olive oil is virgin or extra virgin olive oil.

In a third aspect, the present invention discloses an enzyme preparation for the extraction of olive oil that comprises at least one phospholipase and at least one enzyme that degrades the cell wall. In a preferred embodiment, said phospholipase is selected from the group consisting of phospholipase A1, phospholipase A2, phospholipase A3, phospholipase B, phospholipase C, phospholipase D, phospholipase PL, phosphoinositide phospholipase C, or a mixture thereof. In another preferred embodiment, said enzyme that degrades the cell wall is selected from the group consisting of xylanase, cellulase, pectinase, protease, cutinase, or a mixture thereof.

The concentration of each of the enzymes in the enzyme preparation according to the present invention is determined based on the initial concentration each enzyme is available and the final desired concentration in the mixture to which said enzyme preparation is added during the extraction method. In a preferred embodiment of the present invention, the final concentration in the mixture of the at least one phospholipase is between 0.1 % and 20 % by weight, in a more preferred embodiment, between 1 % and 10 % in weight, in a more preferred embodiment, between 2 % and 5 % by weight.

Hereinafter, the present invention is illustrated by examples, which do not constitute a limitation thereof.

### EXEMPLES

### EXAMPLE 1: Use of enzyme preparations for the extraction of virgin olive oil

Various enzyme preparations containing different types of enzymes were prepared as detailed below. Thus, the enzyme preparations 1 to 3 and the control preparation correspond to preparations known in the state of the art for the extraction of virgin olive oil. On the other hand, enzyme preparations 4 to 12 correspond to the present invention.
- Control preparation: it contained no enzyme, only water.
- Enzyme preparation 1: it contained xylanase, cellulase and pectinase.
- Enzyme preparation 2: contained the enzymes of preparation 1 and further contained protease.
- Enzyme preparation 3: contained the enzymes of preparation 1 and further contained protease of bacterial origin.
- Enzyme preparation 4: it contained phospholipase A1.
- Enzyme preparation 5: it contained phospholipase A2.
- Enzyme preparation 6: it contained phospholipase A3.
- Enzyme preparation 7 contained phospholipase C.
- Enzyme preparation 8: it contained the enzymes of the enzyme preparation 1 and further contained phospholipase A1.
- Enzyme preparation: 9: it contained the enzymes of the enzyme preparation 1 and further contained phospholipase A2.
- Enzyme preparation: 10: it contained the enzymes of the enzyme preparation 1 and further contained phospholipase A3.
- Enzyme preparation: 11: it contained the enzymes of the enzyme preparation 1 and also contained phospholipase C.
- Enzyme preparation: 12: it contained the enzymes of the enzyme preparation 3 and also contained phospholipase A1.
- Enzyme preparation 13: contained phospholipase PL.
- Enzyme preparation 14: it contained phosphoinositide phospholipase C and phospholipase C.
- Enzyme preparation 15: it contained the enzymes of the enzyme preparation 1 and further contained phosphoinositide phospholipase C and phospholipase C.

150 kg of picual type olives from the 2017/2018 campaign previously washed were used in a two-phase extraction method. The olives were crushed using a hammer mill at 3000 rpm to form a paste. The paste was then divided into 15 batches of equal weight and one of the enzyme preparations described above was added to each batch at the beginning of the malaxation step using a 10 % dose of active enzyme protein over the total weight of the paste. Once the malaxation step was completed, the paste was separated into its different components by using a two-phase centrifugal decanter. The oily phase was centrifuged again using a vertical centrifuge. The oil obtained was consequently weighed and analysed using standard techniques of the field.

The yield for each of the procedures was calculated with respect to the initial olive mass. The results are shown in Table 1, where it can be seen how the percentage of oil mass obtained with respect to the original olive mass increases by at least 9.43 % in cases in which at least one phospholipase is used. These results demonstrate that the use of the enzyme preparations of the present invention comprising at least one phospholipase allows to obtain at least 9.43 kg of additional oil for every 100 kg of olives, compared to the methods known in the state of the art.

**Table 1: Yields of the procedures for virgin olive oil extraction.**

| | Oil mass / Olive mass (%) |
|---|---|
| Control | 14,02 |
| Enzyme preparation 1 | 14,51 |
| Enzyme preparation 2 | 15,42 |
| Enzyme preparation 3 | 15,16 |
| Enzyme preparation 4 | 24,00 |
| Enzyme preparation 5 | 23,97 |
| Enzyme preparation 6 | 23,45 |
| Enzyme preparation 7 | 25,28 |
| Enzyme preparation 8 | 24,36 |
| Enzyme preparation 9 | 24,84 |
| Enzyme preparation 10 | 23,87 |
| Enzyme preparation 11 | 25,41 |
| Enzyme preparation 12 | 24,71 |
| Enzyme preparation 13 | 23,58 |
| Enzyme preparation 14 | 25,37 |
| Enzyme preparation 15 | 25,55 |

The results showed that the use of the enzyme preparations containing at least one phospholipase (preparations 4 to 15) allowed to considerably increase the yield of the method for virgin olive oil extraction with respect to the yield of a method in which said phospholipases were not used (control preparation). Thus, the yield went from 14.02 % to yields between 23.45 % and 25.55 %, with the highest yields being observed when the enzyme preparation used included type C phospholipases. In addition, the use of enzymes capable of degrading the cell wall also resulted in the increase of the method yield, as previously described. Thus, a yield of up to 15.42 % was observed when the enzyme preparation contained xylanase, cellulase, pectinase and protease. However, the combined use of said enzymes capable of degrading the cell wall with at least one phospholipase resulted in a much more important yield increase, as it can be seen in the results for preparations 8 to 12 and 15, for which the yields increased to values of around 25.5 %.

These results demonstrate that the use of an enzyme preparation that comprises at least one phospholipase allows to increase the yield of a method for virgin olive oil extraction beyond the increase that can be observed with the use only of enzymes that degrade the cell wall.

### EXAMPLE 2: Comparative study of the properties of a virgin olive oil extracted according to the present invention and a virgin olive oil extracted according to the methods known in the state of the art

The properties of a sample A, consisting of 160 ml of virgin olive oil extracted by using the enzyme preparation 11 of example 1, which contained xylanase, cellulase, pectinase and phospholipase C, and the properties of a sample control B, which consisted of 160 ml of virgin olive oil extracted by the conventional method, i.e., the control in example 1, were analyzed by techniques well known to the person skilled in the art. Results are shown in table 2.

Both samples were negative for the cold test, which confirmed that the steps followed in both methods are within the regulatory framework for obtaining virgin and extra virgin olive oil.

Regarding the quality values of olive oil, as can be seen in Table 2, the acidity level of sample A was 0.9º while it was 0.91º for sample B. Regarding the composition of fatty acids, both samples showed similar profiles, which demonstrated that the use of the enzyme preparation 11 did not modify the composition of the oil. Finally, the triglyceride profile was also not affected by the use of the enzyme preparation of the present invention.

The results obtained revealed that the use of an enzyme preparation comprising at least one phospholipase allows the yield of a method for virgin or extra virgin olive oil extraction to be increased without altering the nutritional and organoleptic properties of said oils.

**Table 2. Comparative analysis of virgin olive oil samples parameters.**

| **Parameter** | **Sample A** | **Sample B (control)** | **Units** | PROCEDURE |
|---|---|---|---|---|
| **Acidity** | 0.90 | 0.91 | % (ac.oleic.) | PNT 1.08 |
| **Peroxide index** | 3.7 | 3.6 | meq O2/kg | PNT 1.09 |

| **Spectrophotometric test** | | | | |
|---|---|---|---|---|
| K 270 | 0.14 | 0.14 | - | PNT 1.10 |
| K 232 | 1.42 | 1.44 | - | PNT 1.10 |
| ΔK | <0.005 | <0.005 | - | PNT 1.10 |
| **Ethyl esters** | 90 | 89 | mg/kg | PNT 1.20 |

| **Fatty Acids Composition** | | | | |
|---|---|---|---|---|
| Lauric (C12:0) | <0.02 | <0.02 | % | PNT 1.14 |
| Myristic (C14:0) | <0.01 | <0.01 | % | PNT 1.14 |
| Palmitic (C16:0) | 10.18 | 10.07 | % | PNT 1.14 |
| Palmitoleic (C16:1) | 0.90 | 0.88 | % | PNT 1.14 |
| Margaric (C17:0) | 0.05 | 0.05 | % | PNT 1.14 |
| Margaroleic (C17:1) | 0.08 | 0.08 | % | PNT 1.14 |
| Stearic (C18:0) | 3.29 | 3.28 | % | PNT 1.14 |
| Oleic (C18:1) | 79.15 | 79.30 | % | PNT 1.14 |
| Linoleic (C18:2) | 4.92 | 4.89 | % | PNT 1.14 |
| Linolenic (C18:3) | 0.66 | 0.65 | % | PNT 1.14 |
| Arachidic (C20:0) | 0.38 | 0.39 | % | PNT 1.14 |
| Eicosenoic (C20:1) | 0.24 | 0.24 | % | PNT 1.14 |
| Behenic (C22:0) | 0.10 | 0.10 | % | PNT 1.14 |
| Erucic(C22:1) | >0.03 | <0.10 | % | PNT 1.14 |
| Lignoceric (C24:0) | 0.05 | 0.05 | % | PNT 1.14 |

| **Total of trans isomers** | | | | |
|---|---|---|---|---|
| Trans oleic (C18:1T) | <0.03 | <0.03 | % | PNT 1.14 |
| Tr L (C18:2T) + Tr Ln (C18:3T) | <0.03 | <0.03 | % | PNT 1.14 |
| Polyphenols (Caffeic) | 426 | 416 | mg Caffeic acid /K | PNT 1.56 (Folin - Ciocalteu) |

| **Triglyceride Profile (complete)** | | | | |
|---|---|---|---|---|
| LLL | 0.05 | 0.04 | % | PNT 1.19 |
| OLLn+PoLL | 0.15 | 0.15 | % | PNT 1.19 |
| PLLn | 0.04 | 0.03 | % | PNT 1.19 |
| OLL | 0.75 | 0.73 | % | PNT 1.19 |
| OOLn+PoOL | 1.29 | 1.29 | % | PNT 1.19 |
| PLL+PoPoO | 0.22 | 0.21 | % | PNT 1.19 |
| POLn+PPoPo+PPoL | 0.53 | 0.51 | % | PNT 1.19 |
| OOL+LnPP | 8.27 | 8.19 | % | PNT 1.19 |
| PoOO | 1.84 | 1.80 | % | PNT 1.19 |
| SLL+PLO | 3.26 | 3.22 | % | PNT 1.19 |
| PoOP+SPoL+SOLn+SPoPo | 0.62 | 0.60 | % | PNT 1.19 |
| PLP | 0.45 | 0.44 | % | PNT 1.19 |
| OOO+PoPP | 49.04 | 49.30 | % | PNT 1.19 |
| SOL | 0.72 | 0.69 | % | PNT 1.19 |
| POO | 22.58 | 22.58 | % | PNT 1.19 |
| POP | 2.62 | 2.64 | % | PNT 1.19 |
| SOO | 6.26 | 6.28 | % | PNT 1.19 |
| POS+SLS | 1.30 | 1.28 | % | PNT 1.19 |

### EXAMPLE 3: Use of enzyme preparations for the extraction of extra virgin olive oil

Two enzyme preparations were prepared according to the present invention, the first of them (A) contained xylanase, cellulase and pectinase and the second (B) further contained phospholipase C.

50 kg of picual type olives from the 2017/2018 campaign previously washed were used in a two-phase extraction method. The olives were crushed using a hammer mill at 3000 rpm to form a paste. The paste was then divided into 3 batches of equal weight and one of the enzyme preparations (A) or (B) or no enzyme preparation (control) was added to each batch at the beginning of the malaxation step using an 8% dose of active enzyme protein over the total weight of the paste. Once the malaxation step was completed, the paste was separated into its different components by using a two-phase centrifugal decanter. The oily phase was centrifuged again using a vertical centrifuge. The oil obtained was consequently weighed and analysed using standard techniques of the field. The results obtained were similar to those of the previous examples.

In this case, the percentage of fat (fatty residue) that was not recovered and therefore remained in the procedure residues, such as oil pomace, was also analyzed. The results obtained are shown in Table 3.

**Table 3: Analysis of the fatty residue obtained in the pomace of a method for extra virgin olive oil extraction.**

| | Fatty residue in the pomace (%) |
|---|---|
| Control | 5,7 |
| Enzyme preparation A | 4,9 |
| Enzyme preparation B | 4,1 |

As can be seen in the results, the fatty residue in the pomace was reduced by the use of the enzyme preparation A that contained enzymes of degradation of the cell wall. However, the combined use of said enzymes with a phospholipase, such as the use of enzyme preparation B, resulted in an even greater reduction in the fat content in the residue, obtaining values of fatty residue in the pomace around 4.1 % compared to 5.7 % obtained in a method wherein no enzymes are used. This reduction of the fatty residue in the pomace directly indicates that the yield of the method for olive oil extraction was more efficient since a lower percentage of the fatty residue was retained in said residue.

### EXAMPLE 4: Use of enzyme preparations in the second pressing of a method for virgin olive oil extraction.

100 kg of picual type olives from the 2018/2019 campaign previously washed were used in a two-phase extraction method. The olives were crushed using a hammer mill at 3000 rpm to form a paste. The paste was then subjected to a first malaxation step and was subsequently separated into its different components by using a two-phase centrifugal decanter. The oily phase was centrifuged again using a vertical centrifuge. The oil obtained was consequently stored and the remains obtained were analysed by near infrared spectrophotometry. Thus, these remains showed a percentage of total fat of 4.13 % and a percentage of fat over dry matter of 10.11 %.

These residues were then subjected to a second step of malaxation and separation in the presence of an enzyme preparation containing xylanase, cellulase, pectinase and phospholipase C at a final concentration for each of them of 8 %, 8.5 %, 7 % and 11 % respectively, over the total mass.

After the second step of malaxation and separation, the properties of the remains were analysed in the same way as previously. The results showed that the percentage of total fat in said remains decreased to 2.03 % and the percentage of fat over dry matter fell to 5.44 %, which implies that the percentage of oil per olive mass was higher, and therefore, the efficiency of the enzyme preparation of the present invention was demonstrated again.

Although the invention has been described with reference to embodiments thereof, it will be understood that these are not limiting the invention, so that multiple constructional details or others that may become evident to the skilled person in the field after interpreting the matter disclosed in the present description and claims. Thus, all variants and equivalents will be included within the scope of the present invention if they can be considered to fall within the broader scope of the following claims.

## Claims

1. Use of an enzyme preparation for the extraction of olive oil, **characterized in that** said enzyme preparation comprises at least one phospholipase

2. Use according to claim 1, **characterized in that** said at least one phospholipase is selected from the group consisting of phospholipase A1, phospholipase A2, phospholipase A3, phospholipase B, phospholipase C, phospholipase D, phospholipase PL, phosphoinositide phospholipase C, or a mixture thereof.

3. Use according to any one of the preceding claims, **characterized in that** said enzyme preparation further comprises at least one enzyme that degrades the cell wall.

4. Use according to claim 3, **characterized in that** said at least one enzyme that degrades the cell wall is selected from the group consisting of xylanase, cellulase, pectinase, protease, cutinase, or a mixture thereof.

5. Use according to any one of the preceding claims, **characterized in that** said enzyme preparation is in the form of a powder or in the form of a glycerol solution.

6. Use according to any one of the preceding claims, **characterized in that** said enzyme preparation is added before or during the grinding step or before or during the malaxation step of a method for olive oil extraction.

7. Use, according to any one of the previous claims, **characterized in that** the olive oil is virgin or extra virgin olive oil.

8. Method for olive oil extraction, **characterized in that** it comprises a step of addition of an enzyme preparation comprising at least one phospholipase.

9. Method according to claim 8, **characterized in that** said at least one phospholipase is selected from the group consisting of phospholipase A1, phospholipase A2, phospholipase A3, phospholipase B, phospholipase C, phospholipase D, phospholipase PL, phosphoinositide phospholipase C, or a mixture thereof..

10. Method according to any one of claims 8 or 9, **characterized in that** said enzyme preparation further comprises at least one enzyme that degrades the cell wall.

11. Method according to claim 10, **characterized in that** said at least one enzyme that degrades the cell wall is selected from the group consisting of xylanase, cellulase, pectinase, protease, cutinase, or a mixture thereof.

12. Method according to any one of claims 8 to 11, **characterized in that** said enzyme preparation is in the form of a powder or in the form of a glycerol solution.

13. Method according to any one of claims 8 to 12, **characterized in that** said step of adding an enzyme preparation comprising at least one phospholipase takes place before or during the grinding step or before or during the malaxation step of said method for olive oil extraction.

14. Method according to any one of claims 8 to 13, **characterized in that** said olive oil is virgin or extra virgin olive oil.

15. Enzyme preparation for the extraction of olive oil **characterized in that** it comprises at least one phospholipase and at least one enzyme that degrades the cell wall selected from the group consisting of xylanase, cellulase, pectinase, protease, cutinase, or a mixture thereof.
